**Europäisches Patentamt**

**European Patent Office**

**Office européen des brevets**

(19)

(11) Veröffentlichungsnummer : **0 461 497 B1**

(12)

# EUROPÄISCHE PATENTSCHRIFT

(45) Veröffentlichungstag der Patentschrift :
**20.10.93 Patentblatt 93/42**

(51) Int. Cl.⁵ : **A61K 7/48,** A61K 7/06,
A61K 31/74, A61L 25/00,
A61K 9/70, A61K 47/32

(21) Anmeldenummer : **91108977.9**

(22) Anmeldetag : **01.06.91**

(54) Verwendung von selektiv hydrierten Styrol-Butadien-Copolymerisaten in kosmetischen und pharmazeutischen Zubereitungen.

(30) Priorität : **13.06.90 DE 4018875**

(43) Veröffentlichungstag der Anmeldung :
**18.12.91 Patentblatt 91/51**

(45) Bekanntmachung des Hinweises auf die
Patenterteilung :
**20.10.93 Patentblatt 93/42**

(84) Benannte Vertragsstaaten :
**DE ES FR GB IT NL**

(56) Entgegenhaltungen :
**EP-A- 0 214 626**
**DE-A- 2 423 849**
**DE-A- 2 712 609**
**DE-A- 3 106 959**
**GB-A- 2 114 580**
**US-A- 4 489 058**

(73) Patentinhaber : **BASF Aktiengesellschaft**
**Carl-Bosch-Strasse 38**
**D-67063 Ludwigshafen (DE)**

(72) Erfinder : **Vogel, Friedrich, Dr.**
**Am Boehlig 13**
**W-6706 Wachenheim (DE)**
Erfinder : **Frosch, Franz, Dr.**
**Auf dem Koeppel 112**
**W-6702 Bad Duerkheim (DE)**
Erfinder : **Westenfelder, Horst**
**Hauptstrasse 126**
**W-7552 Durmersheim (DE)**

## Beschreibung

Die vorliegende Erfindung betrifft die Verwendung von selektiv an der olefinischen Doppelbindung hydrierten Copolymerisaten aus

A) 50 bis 65 Gew.-% Styrol,
B) 35 bis 50 Gew.-% Butadien und
C) 0 bis 5 Gew.-% weiterer copolymerisierbarer Monomerer

in kosmetischen und pharmazeutischen Zubereitungen.

Weiterhin betrifft die Erfindung kosmetische und pharmazeutische Zubereitungen, die diese Copolymerisate enthalten.

Kosmetische und pharmazeutische Zubereitungen wie Hautcremes oder Hautöle haben sehr oft den Nachteil, sich allzu leicht abwaschen zu lassen. Dies ist besonders bei Sonnenschutz-, Baby- und Badekosmetika störend. Die optimale Pflege-, Heil- oder Schutzwirkung der Haut wird nicht voll erreicht und der Verbrauch an diesen Mitteln liegt zu hoch, weil immer wieder neues Mittel aufgetragen werden muß.

Die DE-C 31 06 959 (1) betrifft ein Verfahren zur Herstellung von Copolymerisaten aus 52 bis 54 Gew.-% Styrol und 46 bis 48 Gew.-% Butadien durch Copolymerisation der Monomeren in aliphatischen, cycloaliphatischen oder aromatischen Kohlenwasserstoffen mit Lithiumalkylinitiatoren in Gegenwart von Tetrahydofuran, wobei im Anschluß an die Polymerisation die vom Butadien stammende Doppelbindung des Copolymerisates nach bekannten Verfahren, beispielsweise in Gegenwart von Übergangsmetallkatalysatoren wie Nickel-Aluminium-Katalysatoren, selektiv hydriert wird. Diese Copolymerisate dienen als Viskositätsindexverbesserer für Schmieröle.

Die DE-A 24 23 849 (2) betrifft eine filmbildende Zubereitung für örtliche Anwendung auf der Haut in Form einer Emulsion eines wasserunlöslichen und eines wasserlöslichen filmbildenden Polymers. Als wasserunlösliches Polymer wird u.a. ein Styrol-Butadien-Polymer im Monomergewichtsverhältnis 67:33 genannt. Die Wasserabwaschbarkeit dieses auf die Haut aufgebrachten Films liegt allerdings zu hoch.

Der vorliegenden Erfindung lag daher die Aufgabe zugrunde, kosmetische und pharmazeutische Zubereitungen bereitzustellen, die sich der Haut nur schwer abwaschen lassen.

Demgemäß wurde die Verwendung der eingangs definierten Copolymerisate in kosmetischen und pharmazeutischen Zubereitungen gefunden.

Die verwendeten Copolymerisate sind aus

A) 50 bis 65 Gew.-%, vorzugsweise 52 bis 57 Gew.-% Styrol,
B) 35 bis 50 Gew.-%, vorzugsweise 43 bis 48 Gew.-% Butadien und
C) 0 bis 5 Gew.-% weiterer copolymerisierbarer Monomerer

aufgebaut. Sie werden durch Polymerisation der Komponenten A bis C und anschließende selektive Hydrierung an der olefinischen Doppelbindung hergestellt. Bevorzugt wird die Verwendung von Copolymerisaten aus den Komponenten A und B allein.

Die Polymerisation und die Hydrierung werden zweckmäßigerweise nach dem in (1) beschriebenen Verfahren durchgeführt. Nach der Hydrierung sollten mindestens 95 %, vorzugsweise mindestens 97 % der olefinischen Doppelbindungen und maximal 5 %, vorzugsweise maximal 3 % der aromatischen Ungesättigtheiten hydriert vorliegen.

Bei den hergestellten Copolymerisaten handelt es sich um statistische Polymere. Sie haben in der Regel ein Molekulargewicht im Bereich zwischen 75 000 und 130 000.

Als weitere copolymerisierbare Monomere C, mit welchen die Eigenschaften der Copolymerisate modifiziert werden können, dienen beispielsweise Methylstyrol, Isopren, Chloropren, Isobutylen, Ethylen, Acrylnitril, Alkylacrylate und -methacrylate, Vinylacetat, Vinylchlorid, Vinylidenchlorid oder Vinylalkylether.

In Frage kommende kosmetische Zubereitungen sind vor allem Hautcremes wie Pflegecremes, Babycremes, Handschutzcremes oder Sonnenschutzcremes, Hautöle wie Badeöle, Babyöle oder Sonnenschutzöle, Salben, Lotionen oder Schminken. Pharmazeutische Zubereitungen wie Salben oder Cremes dienen zur Applikation von Wirkstoffen. In den genannten kosmetischen und pharmazeutischen Zubereitungen kann es sich sowohl um Wasser-in-Öl- als auch um Öl-in-Wasser-Emulsion oder um eine Kombination beider Typen handeln.

Gegenstand der vorliegenden Erfindung sind weiterhin kosmetische und pharmazeutische Zubereitungen, die die beschriebenen Copolymerisate in einer Menge von 1 bis 20 Gew.-%, vorzugsweise 2 bis 10 Gew.-%, bezogen auf die Gesamtmenge der Zubereitung, enthalten.

Die üblichen Zusammensetzungen und Bestandteile sowie übliche Emulgatoren, Hilfsmittel und Zusatzstoffe wie Stabilisatoren oder Konservierungsmittel für derartige kosmetische und pharmazeutische Zubereitungen sind dem Fachmann bekannt und brauchen deshalb hier nicht näher erläutert zu werden.

Die die erfindungsgemäß verwendeten Copolymerisate enthaltenden kosmetischen und pharmazeutischen Zubereitungen zeichnen sich dadurch aus, daß es sich sowohl mit klarem als auch mit oberflächenaktive Mittel enthaltendem Wasser nur schwer wieder abwaschen lassen und somit auf der Haut ihre Pflege-, Heil- oder Schutzwirkung voll entfalten können. Außerdem weisen die erfindungsgemäß verwendeten Copolymerisate eine deutliche Schutzwirkung für die Haut auf, da sie das Eindringen von schädigenden Substanzen erschweren.

Weiterhin wirken die erfindungsgemäß verwendeten Copolymerisate verdickend auf die sie enthaltenden Zubereitungen, ersichtlich an den höheren Viskositätswerten, wodurch die Verarbeitung, Lagerung und Applikation der Zubereitungen vereinfacht werden kann, weil man mit Hilfe der Copolymerisate die gewünschten Viskositätsbereiche besser einstellen kann.

Beispiel 1

Abwaschbarkeit von kosmetischen Creme-Zubereitungen von der Haut

Ein Copolymerisat, welches gemäß (1) durch Polymerisation von 53 Gew.-% Styrol mit 47 Gew.-% Butadien mit sek.-Butyllithium als Katalysator in einer Cyclohexan-Tetrahydrofuran-Mischung und anschließende selektive Hydrierung mit Wasserstoff in Gegenwart von Nickel(II)-acetylacetonat/Aluminiumtriisobutyl als Hydrierungskatalysator hergestellt worden war, wurde in kosmetische Zubereitungen eingearbeitet. Diesen Zubereitungen wurde zur visuellen Beurteilung der Abwaschbarkeit von der Haut ein Farbstoff (Methylenblau oder Sudanrot) zugemischt.

11 Testpersonen erhielten jeweils eine 0,5 g-Probe der Zubereitung mit dem erfindungsgemäß verwendeten Copolymerisat und eine 0,5 g-Vergleichsprobe, wobei aber nicht bekannt war, welches die erfindungsgemäße Probe und welches die Vergleichsprobe war. Die Proben wurden auf die beiden frisch gewaschenen Handrücken aufgetragen, danach wurden die Handrücken 10 sec mit 30°C warmem Wasser abgebraust und anschließend wurden die Intensitäten der auf den beiden Handrücken verbliebenen Färbungen gegeneinander verglichen.

Im Anschluß daran wuschen sich alle Testpersonen ihre Handrücken mit Wasser mit einem Waschmittelzusatz, nämlich mit einer 10 gew.-%igen wäßrigen Natriumlaurylethersulfat-Lösung, durch 5 sec dauerndes gleichmäßiges Reiben und spülten danach die Waschlösung mit 30°C warmem Wasser kurz ab. Die Intensitäten der Färbungen auf beiden Handrücken wurden erneut gegeneinander verglichen.

Die Proben wiesen folgende Zusammensetzungen auf:

Probe 1 (erfindungsgemäß):

Wasser-in-Öl Creme mit Methylenblau (Viskosität: 10 000 mPa·s)

Phase I:

| | |
|---|---|
| 4,0 Gew.-% | mit 7 mol Ethylenoxid umgesetztes hydriertes Rizinusöl |
| 3,0 Gew.-% | einer Mischung aus Polyglyceryl-2-sesquiisostearat, Bienenwachs, Mineralöl, Magnesiumstearat und Aluminiumstearat |
| 2,0 Gew.-% | mikrokristallines Wachs |
| 0,5 Gew.-% | einer Mischung aus Mineralöl und Lanolinalkohol |
| 8,0 Gew.-% | Isopropylmyristat |
| 8,0 Gew.-% | Polypropylenglycol-3-myristylether |
| 4,0 Gew.-% | Cetyl-stearyl-2-ethylhexanoat |
| 4,0 Gew.-% | Paraffinöl |
| 3,0 Gew.-% | Polyethylenglycol-Dodecylglycol-Copolymer |
| 3,0 Gew.-% | Hydroxyoctacosanyl-hydroxystearat |
| 3,0 Gew.-% | 2-Hydroxy-4-methoxybenzophenon |
| 1,0 Gew.-% | Aluminiumstearat |
| 4,0 Gew.-% | des Styrol-Butadien-Copolymerisates |

Phase II:

| | |
|---|---|
| 5,0 Gew.-% | 1,2-Propylenglykol |
| 3,0 Gew.-% | eines mit 25 mol Ethylenoxid umgesetzten p-Aminobenzoesäureethylesters |
| 0,2 Gew.-% | Methyl- und Propylparaben |
| 0,3 Gew.-% | Imidazolidinylharnstoff |
| 43,5 Gew.-% | Wasser |
| 0,5 Gew.-% | Methylenblau |

Probe A (zum Vergleich):

Wasser-in-Öl-Creme mit Methylenblau (Viskosität: 600 mPa·s)
    wie Probe 1, nur ohne Styrol-Butadien-Copolymerisat und mit 8,0 Gew.-% anstelle von 4,0 Gew.-% Cetyl-stearyl-2-ethylhexanoat.

Probe 2 (erfindungsgemäß):

Wasser-in-Öl-Creme mit Sudanrot (Viskosität: 18 000 mPa·s)
    wie Probe 1, nur mit 0,5 Gew.-% Sudanrot in Phase I anstelle von 0,5 Gew.-% Methylenblau in Phase II.

Probe B (zum Vergleich):

Wasser-in-Öl-Creme mit Sudanrot (Viskosität: 700 mPa·s)
    wie Probe 2, nur ohne Styrol-Butadien-Copolymerisat und mit 8,0 Gew.-% anstelle von 4,0 Gew.-% Cetyl-stearyl-2-ethylhexanoat.

Probe 3 (erfindungsgemäß):

Öl-in-Wasser-Creme mit Sudanrot (Viskosität: 3 000 mPa·s)
        Phase I:
    1,0 Gew.-%      einer Mischung aus mit 6 mol Ethylenoxid umgesetztem Cetyl-stearylalkohol und Stearylalkohol
    1,0 Gew.-%      mit 25 mol Ethylenoxid umgesetzter Cetyl-stearylalkohol
    3,0 Gew.-%      Glycerylstearat
    0,2 Gew.-%      (-)-2-Bisabolol
    5,0 Gew.-%      Paraffinöl
    10,0 Gew.-%     Benzoesäure-$C_{12}$-$C_{15}$-alkylester
    5,0 Gew.-%      Jojobaöl
    0,2 Gew.-%      $\alpha$-Tocopherol
    2,0 Gew.-%      Cetyl-stearylalkohol
    3,0 Gew.-%      2-Hydroxy-4-methoxybenzophenon
    5,0 Gew.-%      Cetyl-stearyl-2-ethylhexanoat
    4,0 Gew.-%      des Styrol-Butadien-Copolymerisates
    0,5 Gew.-%      Sudanrot
        Phase II:
    3,0 Gew.-%      1,2-Propylenglykol
    2,0 Gew.-%      2-Hydroxy-4-methoxybenzophenon-5-sulfonsäure
    1,0 Gew.-%      Triethanolamin
    0,3 Gew.-%      Imidazolidinylharnstoff
    0,2 Gew.-%      Methyl- und Propylparaben
    53,6 Gew.-%     Wasser

Probe C (zum Vergleich):

Öl-in-Wasser-Creme mit Sudanrot (Viskosität: 4 500 mPa·s)
    wie Probe 3, nur ohne Styrol-Butadien-Copolymerisat und mit 9,0 Gew.-% anstelle von 5,0 Gew.-% Cetyl-stearyl-2-ethylhexanoat.

    Aus Tabelle 1 sind die Ergebnisse der Tests ersichtlich. Angegeben sind jeweils für die Abwaschbarkeit mit reinem Wasser und mit Wasser mit Waschmittelzusatz die Anzahl der Beurteilungen, bei denen die Färbung mit der erfindungsgemäßen Probe gegenüber der Färbung mit der Vergleichsprobe nach dem Waschen als deutlich intensiver eingestuft wurde, ohne Kenntnis, welches die erfindungsgemäße Probe und welches die Vergleichsprobe war.

Tabelle 1: Anzahl der Beurteilungen

| Probe | gegen | Vergleichsprobe | Abwaschbarkeit mit | |
|-------|-------|-----------------|--------------------|---|
| | | | reinem Wasser | Wasser mit Wasch- mittelzusatz |
| 1 | | A | 10 von 11 | 9 von 11 |
| 2 | | B | 10 von 11 | 10 von 11 |
| 3 | | C | 5 von 11 | 4 von 11 |

Beispiel 2

Hautschutzwirkung von Paraffinöl-Zubereitungen

Zur Beurteilung der Hautschutzwirkung wurde eine 8 gew.-%ige Zubereitung des Styrol-Butadien-Copolymerisates aus Beispiel 1 in Paraffinöl hergestellt (Probe 4). Als Vergleich diente Paraffinöl, das 8 Gew.-% hochdisperse Kieselsäure als Verdickungsmittel enthielt, um eine vergleichbare Viskosität aufzuweisen (Probe D).

Die beiden Unterarme von 5 Testpersonen wurden mit einer 1 gew.-%igen wäßrigen Bromthymolblau-Lösung bestrichen und danach mit der Probe auf dem einen und der Vergleichsprobe auf dem anderen Arm überdeckt. Die so behandelten Hautflächen wurde mit 0,1 normaler wäßriger Natronlauge in Kontakt gebracht. Das Durchdringen der Paraffinöl-Zubereitung ließ sich durch die Blaufärbung des Indikatorfarbstoffes verfolgen.

Tabelle 2 zeigt die Zeiten bis zur beginnenden Blaufärbung und bis zur Durchfärbung bei Verwendung der erfindungsgemäßen Probe 4 und der Vergleichsprobe D.

Tabelle 2: Zeiten bis zur Blaufärbung

| Testperson | Zeit [sec] bis zur | |
|------------|--------------------|---|
| | beginnenden Blaufärbung | Durchfärbung |
| **Probe 4:** | | |
| 1 | 35 | > 120 |
| 2 | 35 | > 120 |
| 3 | 50 | > 120 |
| 4 | 65 | > 120 |
| 5 | 40 | > 120 |
| **Vergleichsprobe D:** | | |
| 1 | 10 | 50 |
| 2 | 15 | 60 |
| 3 | 12 | 60 |
| 4 | 35 | 120 |
| 5 | 15 | 60 |

**Patentansprüche**

**Patentansprüche für folgende Vertragsstaaten : DE, FR, GB, IT, NL**

1.  Verwendung von selektiv an der olefinischen Doppelbindung hydrierten Copolymerisaten aus
    A) 50 bis 65 Gew.-% Styrol,
    B) 35 bis 50 Gew.-% Butadien und
    C) 0 bis 5 Gew.-% weiterer copolymerisierbarer Monomerer
    in kosmetischen und pharmazeutischen Zubereitungen.

2.  Verwendung von Copolymerisaten nach Anspruch 1 aus
    A) 52 bis 57 Gew.-% Styrol,
    B) 43 bis 48 Gew.-% Butadien und
    C) 0 bis 5 Gew.-% weiterer copolymerisierbarer Monomerer.

3.  Verfahren zur Herstellung von kosmetischen und pharmazeutischen Zubereitungen, dadurch gekennzeichnet, daß man hierzu Copolymerisate gemäß Anspruch 1 oder 2 verwendet.

4.  Kosmetische und pharmazeutische Zubereitungen, enthaltend 1 bis 20 Gew.-%, bezogen auf die Gesamtmenge der Zubereitung, eines oder mehrerer Copolymerisate gemäß Anspruch 1 oder 2.

**Patentansprüche für folgenden Vertragsstaat : ES**

1.  Verfahren zur Herstellung von kosmetischen und pharmazeutischen Zubereitungen, dadurch gekennzeichnet, daß man hierfür übliche Bestandteile mit selektiv an der olefinischen Doppelbindung hydrierten Copolymerisaten aus
    A) 50 bis 65 Gew.-% Styrol,
    B) 35 bis 50 Gew.-% Butadien und
    C) 0 bis 5 Gew.-% weiterer copolymerisierbarer Monomerer
    mischt.

2.  Verfahren zur Herstellung von kosmetischen und pharmazeutischen Zubereitungen nach Anspruch 1, dadurch gekennzeichnet, daß man hierbei Copolymerisate aus
    A) 52 bis 57 Gew.-% Styrol,
    B) 43 bis 48 Gew.-% Butadien und
    C) 0 bis 5 Gew.-% weiterer copolymerisierbarer Monomerer
    verwendet.

**Claims**

**Claims for following Contracting States : DE, FR, GB, IT, NL**

1.  The use of copolymers which are selectively hydrogenated on the olefinic double bond and are composed of
    A) 50 to 65 % by weight styrene,
    B) 35 to 50 % by weight butadiene and
    C) 0 to 5 % by weight other copolymerizable monomers
    in cosmetic and pharmaceutical compositions.

2.  The use of copolymers as claimed in claim 1, composed of
    A) 52 to 57 % by weight styrene,
    B) 43 to 48 % by weight butadiene and
    C) 0 to 5 % by weight other copolymerizable monomers.

3.  A process for preparing cosmetic and pharmaceutical compositions, which comprises using for this purpose copolymers as claimed in claim 1 or 2.

4.  A cosmetic or pharmaceutical composition containing 1 to 20 % by weight, based on the total weight of the composition, of one or more copolymers as claimed in claim 1 or 2.

**Claims for the following Contracting State : ES**

1. A process for preparing cosmetic and pharmaceutical compositions, which comprises mixing conventional ingredients therefor with copolymers which are selectively hydrogenated on the olefinic double bond and are composed of
   A) 50 to 65 % by weight styrene,
   B) 35 to 50 % by weight butadiene and
   C) 0 to 5 % by weight other copolymerizable monomers.

2. A process for preparing cosmetic and pharmaceutical compositions as claimed in claim 1, which comprises using for this copolymers composed of
   52 to 57 % by weight styrene,
   43 to 48 % by weight butadiene and
   0 to 5 % by weight other copolymerizable monomers.

**Revendications**

**Revendications pour les Etats contractants suivants : DE, FR, GB, IT, NL**

1. Utilisation de copolymères hydrogénés sélectivement sur la double liaison oléfinique, à base de
   A) 50 à 65% en poids de styrène,
   B) 35 à 50% en poids de butadiène et
   C) 0 à 5% en poids d'autres monomères copolymérisables,
   dans des compositions cosmétiques et pharmaceutiques.

2. Utilisation selon la revendication 1 de copolymères à base de
   A) 52 à 57% en poids de styrène,
   B) 43 à 48% en poids de butadiène et
   C) 0 à 5% en poids d'autres monomères copolymérisables.

3. Procédé de préparation de compositions cosmétiques et pharmaceutiques, caractérisé en ce qu'on utilise à cette fin des copolymères selon la revendication 1 ou 2.

4. Compositions cosmétiques et pharmaceutiques, contenant de 1 à 20% en poids, par rapport à la quantité totale de la composition, d'un ou de plusieurs copolymères selon la revendication 1 ou 2.

**Revendications pour l'Etat contractant suivant : ES**

1. Procédé de préparation de compositions cosmétiques et pharmaceutiques, caractérisé en ce qu'on mélange à cet effet des constituants usuels avec des copolymères hydrogénés sélectivement sur la double liaison oléfinique, à base de
   A) 50 à 65% en poids de styrène,
   B) 35 à 50% en poids de butadiéne et
   C) O à 5% en poids d'autres monomères copolymérisables.

2. Procédé de préparation de compositions cosmétiques et pharmaceutiques selon la revendication 1, caractérisé en ce qu'on y utilise des copolymères à base de
   A) 52 à 57% en poids de styrène,
   B) 43 à 48% en poids de butadiène et
   C) 0 à 5% en poids d'autres monomères copolymérisables.